# EUROPÄISCHE PATENTSCHRIFT

(11) **EP 3 187 160 B1**
(45) Veröffentlichungstag und Bekanntmachung des Hinweises auf die Patenterteilung: **25.07.2018**
(21) Anmeldenummer: 16200614.2
(22) Anmeldetag: 25.11.2016
(51) Int. Cl.: A61F 5/01, A61F 13/00, A61F 13/08

(54) **KOMPRESSIONSBANDAGE ZUR BEHANDLUNG VON LYMPHÖDEMEN**
COMPRESSION BANDAGE FOR THE TREATMENT OF LYMPHEDEMA
BANDAGE DE COMPRESSION POUR LE TRAITEMENT DE LYMPHOEDÈMES

(30) Priorität: 30.12.2015 DE 202015107145 U
(43) Veröffentlichungstag der Anmeldung: 05.07.2017
(73) Patentinhaber: Julius Zorn GmbH, 86551 Aichach (DE)
(72) Erfinder: Schettler, Uwe, 86551 Aichach (DE)
(74) Vertreter: Charrier Rapp & Liebau

(56) Entgegenhaltungen:
- EP-B1- 1 735 019
- WO-A1-2011/066237
- WO-A1-2014/160572
- US-A1- 2005 131 321

## Beschreibung

Die Erfindung betrifft eine Kompressionsbandage zur Behandlung von Lymphödemen nach dem Oberbegriff des Anspruchs 1.

Lymphödeme sind Flüssigkeitsansammlungen im Zwischenzellraum, die durch mechanische Insuffizienz des Lymphgefäßsystems des menschlichen oder tierischen Körpers hervorgerufen werden. Ursache kann ein chronisch- entzündliches Krankheitsbild sein, das vor allem die Körperextremitäten betrifft. Wegen der mechanischen Insuffizienz kann die Körperflüssigkeit im Zwischenzellraum nicht mehr ausreichend über die Lymphgefäße abtransportiert werden, was zu einem Rückstau und zur Ansammlung von Flüssigkeit in den Zellzwischenräumen führt.

Es ist aus dem Stand der Technik bekannt, Lymphödeme durch Kompression der betroffenen Körperextremitäten zu behandeln. Hierfür werden bspw. Kompressionsbandagen um die betroffenen Arme oder Beine gewickelt, um einen externen Kompressionsdruck auszuüben. Der Kompressionsdruck unterstützt den Abtransport der Lymphflüssigkeit und dadurch den Abbau des Lymphstaus.

Aus der EP 1 735 019B1 ist eine Kompressionsbandage zur Behandlung von Lymphödemen bekannt, welche ein erstes und ein zweites Band umfasst, die jeweils um einen Abschnitt der zu behandelnden Körperextremität geschlungen und mittels Befestigungselemente so festgelegt werden, dass sie einen vorgegeben Kompressionsdruck auf die Körperextremität ausüben. Die beiden Bänder sind dabei aneinander befestigt und umfassen Kurzugbänder, die einen maximalen elastomeren Dehnungsbereich von 15% bis zu 100% eines ungedehnten Zustandes aufweisen und einen Kompressionsdruck in vorgegebenen Druckbereichen auf die Körperextremität ausüben, wenn sie an der Körperextremität bei maximaler Dehnung um die Extremität gewickelt werden. Das gewünschte Maß an Kompression eines Bandes wird dabei durch Variieren der Länge und Breite des Bandes und/oder der Zusammensetzung des Bandes erhalten. Die Kurzzugbänder umfassen dabei ein Stützmaterial und ein elastomeres Material.

Eine weitere Kompressionsbandage zur Behandlung von Lymphödemen ist aus der US 7 329 232 B2 bekannt, wobei auch diese Kompressionsbandage mehrere Bänder umfasst, die sich von einem flachen Zentralbereich erstrecken und um eine zu behandelnde Körperextremität gewickelt und durch Befestigungselemente auf dem flachen Zentralbereich der Bandage befestigt werden können, um bei angelegter Bandage einen Kompressionsdruck auf die Körperextremität auszuüben.

Aus der WO2011/066237 A1 ist ein Bekleidungsstück zur therapeutischen Kompressionsbehandlung mit einem Grundkörper, und einem Rückenkörper bekannt, wobei der Rückenkörper am Grundkörper abnehmbar befestigbar ist und sowohl der Grundkörper als auch der Rückenkörper eine Mehrzahl von abstehenden Bändern aufweist, um den Grundkörper und den Rückenkörper einander zu befestigen, wenn das Bekleidungsstück um eine Körperextremität geschlungen ist. Eine weitere Bandage zur Kompressionstherapie von Körperextremitäten des menschlichen Körpers ist aus der US2005/0131321 A1 bekannt. Diese Bandage umfasst einen dehnbaren Körper und eine aufblasbare Kammer, wobei der dehnbare Körper um die Körperextremität gelegt wird, um die Aufblaskammer an der Körperextremität anzuordnen.

Ein gewünschter Kompressionsdruck kann bei den bekannten Kompressionsbandagen durch unterschiedliche Dehnung der Bänder erzeugt und durch Befestigung der Bänder in einer bestimmten Position eingestellt werden. Da jedes der Bänder einen durch das Material des Bandes vorgegebenen Dehnungsbereich mit einer maximalen Dehnung, im Vergleich zum ungedehnten Zustand, aufweist, ist jedoch der erzielbare Bereich des Kompressionsdrucks durch die Dehnungscharakteristik der einzelnen Bänder vorgegeben und beschränkt.

Bei den bekannten Kompressionsbandagen kann es weiterhin dazu kommen, dass das durch die Lymphinsuffizienz angeschwollene Körpergewebe der Extremität zwischen benachbarten Bändern, die um unterschiedliche Abschnitte der Körperextremität gewickelt sind, herausquellen kann, was zu einem verminderten therapeutischen Effekt der Kompressionsbandage führt.

Hiervon ausgehend liegt der Erfindung die Aufgabe zugrunde, eine Kompressionsbandage zur Behandlung von Lymphödemen bereitzustellen, mit der ein weiter Bereich von Kompressionsdrücken erzielbar ist und das Herausquellen von angeschwollenem Körpergewebe der zu behandelnden Körperextremität weitgehend unterbunden werden kann.

Diese Aufgaben werden mit einer Kompressionsbandage zur Behandlung von Lymphödemen mit den Merkmalen des Anspruchs 1 gelöst. Bevorzugte Ausführungsbeispiele der erfindungsgemäßen Kompressionsbandage sind den abhängigen Ansprüchen zu entnehmen.

Die erfindungsgemäße Kompressionsbandage gemäß Anspruch 1 umfasst einen Bandagenkörper aus einem elastischen Material zum Umschlingen der zu behandelnden Körperextremität, mit einer Innenseite und einer Außenseite sowie einem ersten und einem zweiten Endabschnitt des Bandagenkörpers, sowie eine erste Gruppe von Befestigungslaschen und eine zweite Gruppe von Befestigungslaschen, wobei die erste Gruppe von Befestigungslaschen an einem Endabschnitt des Bandagenkörpers angeordnet ist und der andere Endabschnitt eine laschenfreie Außenkante aufweist und die erste Gruppe von Befestigungslaschen eine Mehrzahl von Befestigungslaschen umfasst, die zweckmäßig eng aneinander angrenzen, durch Einschnitte in dem Bandagenkörper voneinander getrennt sind und jeweils über erste Befestigungsmittel verfügen, über welche die jeweilige Befestigungslasche der ersten Gruppe von Befestigungslaschen an der Außenseite des Bandagenkörpers befestigbar ist. Die zweite Gruppe von Befestigungslaschen umfasst dabei ebenfalls eine Mehrzahl von Befestigungslaschen, die zweckmäßig aneinander angrenzen, durch Einschnitte voneinander getrennt sind und auf der Außenseite des Bandagenkörpers so befestigt werden können, dass sie die Einschnitte zwischen den Befestigungslaschen der ersten Gruppe überdecken, wenn die Kompressionsbandage an der Körperextremität angelegt ist.

Bei dieser Kompressionsbandage gemäß der Erfindung ist es durch die erste Gruppe von Befestigungslaschen und die zweite Gruppe von Befestigungslaschen möglich, den von der Kompressionsbandage auf die Körperextremität ausgeübten Kompressionsdruck in weiten Bereichen zu variieren, indem zunächst mittels der ersten Befestigungslaschen ein vorgegebener Kompressionsdruck erzeugt und dieser durch die Befestigungslaschen der zweiten Gruppe noch variiert wird, indem mit den Befestigungslaschen der zweiten Gruppe eine zusätzliche Dehnung des Bandagenkörpers und damit eine noch höhere Kompression auf die Körperextremität erzeugt wird. Die Befestigungslaschen der ersten Gruppe und der zweiten Gruppe werden dabei jeweils über erste bzw. zweite Befestigungsmittel bei um die Körperextremität geschlungenem Bandagenkörper an der Außenseite des Bandagenkörpers befestigt. Bei angelegter Kompressionsbandage überdecken die Befestigungslaschen der zweiten Gruppe die Befestigungslaschen der ersten Gruppe im Bereich der Einschnitte, welche die Befestigungslaschen der ersten Gruppe voneinander trennt. Dadurch wird verhindert, dass angeschwollenes Körpergewebe zwischen benachbarten Befestigungslaschen der ersten Gruppe durchquellen kann. Bevorzugt sind die Befestigungslaschen der zweiten Gruppe in Längsrichtung des Bandagenkörpers versetzt zu den ersten Befestigungslaschen angeordnet, so dass die Einschnitte zwischen benachbarten Befestigungslaschen der ersten Gruppe jeweils durch eine der Befestigungslaschen der zweiten Gruppe überdeckt werden. Auf diese Weise kann durch die von den Einschnitten gebildeten Zwischenräume kein Körpergewebe durchquellen und die Kompressionsbandage erzeugt vollumfänglich einen weitgehend gleichmäßigen Kompressionsdruck auf die Körperextremität.

Zweckmäßig umfassen die Befestigungsmittel der Befestigungslaschen der ersten und der zweiten Gruppe Klettverschlüsse, wobei Hakenteile der Klettverschlusse an einem äußeren Ende jeder Befestigungslasche der ersten und zweiten Gruppe angeordnet sind und die Außenseite des Bandagenkörpers mit zu den Hakenteilen korrespondierenden Schlaufen zum Anhaften der Hakenteile ausgebildet ist.

Der Bandagenkörper und zweckmäßig zumindest ein Teilbereich der Befestigungslaschen ist aus einem elastischen Material gebildet, insbesondere aus einem Kurzzugmaterial mit einem Dehnungsbereich zwischen 15% und 50% des ungedehnten Zustands. Der bei angelegter Kompressionsbandage auf die Körperextremität ausgeübte Kompressionsdruck kann damit zweckmäßig im Bereich zwischen 8mmHg und über 40 mmHg bis hin zu 60 mmHg eingestellt werden, indem der Bandagenkörper durch Zug an den Befestigungslaschen gedehnt und die Befestigungslaschen in einer gewünschten Position auf der Außenseite des Bandagenkörpers befestigt werden.

Um das Anlagen der Kompressionsbandagen an einer Körperextremität, bspw. einem Bein oder einem Arm, zu erleichtern, weist die Kompressionsbandage eine Anziehhilfe in Form eines schlauchförmigen Einsatzes auf, der an der Innenseite des Bandagenkörpers angeordnet ist.

Der schlauchförmige Einsatz kann dabei von einem schlauchförmigen elastischen Textilmaterial gebildet sein, welches an der Innenseite des Bandagenkörpers befestigt, insbesondere angenäht ist. Die Anziehhilfe kann auch durch ein an der Innenseite des Bandagenkörpers befestigtes, insbesondere angenähtes Tuch aus einem elastischen Material gebildet sein, welches so an der Innenseite des Bandagenkörpers befestigt ist, dass zwischen der Innenseite des Tuchs und der Innenseite des Bandagenkörpers ein schlauchförmiger Zwischenraum entsteht, durch den die zu behandelnde Körperextremität beim Anlagen der Kompressionsbandage durchgeführt werden kann.

Zum Anlegen der Kompressionsbandage mit Anziehhilfe wird die zu behandelnde Körperextremität zunächst durch den schlauchförmigen Einsatz durchgeführt und danach wird der Bandagenkörper eng anliegend um die Körperextremität geschlungen. Anschließend werden die Befestigungslaschen der Gruppe von ersten Befestigungslaschen der Reihe nach, bspw. von proximal nach distal, zuerst manuell gezogen, so dass sie den Bandagenkörper dehnen und danach werden die ersten Befestigungslaschen jeweils mit ihren ersten Befestigungsmitteln auf der Außenseite des Bandagenkörpers befestigt, indem bspw. die Hakenteile am Endabschnitt jeder Befestigungslasche auf die Außenseite des Bandagenkörpers gelegt und dort angeheftet werden. Auf diese Weise kann durch den von den ersten Befestigungslaschen auf den Bandagenkörper ausgeübte Zug eine bestimmte Kompression des Bandagenkörpers auf die innenliegende Körperextremität erzeugt werden. Erforderlichenfalls können die Befestigungslaschen der ersten Gruppe noch nachgezogen werden, indem die Befestigung am Bandagenkörper wieder kurzzeitig gelöst und an einer anderen Stelle wieder fest gelegt wird, an der die jeweilige Befestigungslasche den Bandagenkörper noch weiter dehnt.

Um den von den Befestigungslaschen der ersten Gruppe ausgeübten Kompressionsdruck noch weiter zu erhöhen, werden die Befestigungslaschen der zweiten Gruppe ebenfalls der Reihe nach, bspw. von proximal nach distal, zunächst gezogen, um den Bandagenkörper noch weiter zu dehnen, und danach mit den zweiten Befestigungsmitteln ebenfalls auf der Außenseite des Bandagenkörpers befestigt, bspw. mittels der Hakenteile der Klettverschlüsse, welche an den Endbereichen der Befestigungslaschen der zweiten Gruppe angeordnet sind, und schließlich auf die flauschige Außenseite des Bandagenkörpers gelegt und dort angeheftet.

Ein besonders einfaches und schnelles Befestigen und Lösen der Befestigungslaschen wird ermöglicht, wenn die ersten Befestigungsmittel und/oder die zweiten Befestigungsmittel Klettverschlüsse umfassen, mit denen die Befestigungslaschen an der Außenseite des Bandagenkörpers befestigt werden können, wobei die Klettverschlüsse dabei zweckmäßig Hakenteile aufweisen, welche insbesondere im Bereich der freien Enden an den Befestigungslaschen befestigt sind und mit Schlaufen auf der Außenseite des Bandagenkörpers zur Herstellung einer Klettverbindung zusammen wirken. Die Klettverschlüsse ermöglichen es, die freien Enden der Befestigungslaschen an jeder beliebigen Stelle auf der Außenseite des Bandagenkörpers zu befestigen.

Durch die Befestigungslaschen der ersten und zweiten Gruppe, die schließlich im angelegten Zustand der Kompressionsbandage übereinander liegen, ist es möglich, den von der Kompressionsbandage auf die innenliegende Körperextremität ausgeübten Kompressionsdruck zu variieren und auf einen gewünschten Wert spezifisch und individuell an die Bedürfnisse des Patienten angepasst einzustellen. Durch das Übereinanderlegen der Befestigungslaschen der ersten und der zweiten Gruppe wird dabei verhindert, dass angeschwollenes Körpergewebe durch die von Einschnitten im Bandagenkörper gebildete Zwischenräume zwischen benachbarten Befestigungslaschen der ersten Gruppe durchtreten kann, da diese Zwischenräume von einer Befestigungslasche der zweiten Gruppe überdeckt werden.

Um zu verhindern, dass die Kompressionsbandage nach dem Anlagen an der Körperextremität verrutschen oder sich bei Bewegungen der Körperextremität wulstförmig aufrollen kann, ist an dem Bandagenkörper ein Stabilisierungselement angeordnet. Das Stabilisierungselement kann bspw. durch zwei an der Außenseite des Bandagenkörpers aufgenähte, insbesondere bogenförmige Rippen gebildet sein, die sich zweckmäßig V- oder X-förmig kreuzen. Eine V- oder X-förmige Kreuzung der beiden Rippen ist besonders bei Kompressionsbandagen für den Unterschenkelbereich vorteilhaft, denn die Form und die Anordnung der bogenförmigen und V- oder X-förmig gekreuzten Rippen können dann anatomisch an die Form der Wade angepasst werden, so dass die beiden Rippen im Bereich des V bzw. im oberen Bereich der X-Kreuzung die Wade umschließen. Die bogenförmigen Rippen können bspw. aus einem Textil- oder einem Kunststoffmaterial gebildet sein, welches auf der Außenseite des Bandagenkörpers aufgenäht ist. Die Rippen stabilisieren dabei den Bandagenkörper im angelegten Zustand der Kompressionsbandage an der Körperextremität und verhindern, dass sich die Kompressionsbandage wulstförmig aufrollen kann, bspw. ausgehend vom Kniekehlenbereich bei einer Abwinklung des Unterschenkels.

Zur anatomischen Anpassung der Kompressionsbandage an die Form der Körperextremität, an welche die Kompressionsbandage angelegt werden soll, ist es zweckmäßig, wenn der Bandagenkörper in einem Zentralbereich eine Wölbung aufweist. Eine solche Wölbung kann bspw. bei einer Kompressionsbandage für einen Unterschenkel den konvex gewölbten Wadenbereich des Unterschenkels aufnehmen und ist dadurch gut an die Anatomie der Körperextremität angepasst. Auch bei Kompressionsbandagen, die für eine Körperextremität mit einem Gelenk (bspw. für einen Arm) vorgesehen sind, kann eine entsprechende Wölbung im Zentralbereich des Bandagenkörpers vorteilhaft sein, um das Gelenk der Körperextremität, (bspw. das Armgelenk) aufnehmen zu können, so dass die Beweglichkeit des Gelenks durch die angelegte Kompressionsbandage nicht beeinträchtigt wird.

Die Herstellung der Kompressionsbandage wird vereinfacht, wenn der Bandagenkörper aus zwei Teilen zusammen gesetzt ist, welche entlang einer Längsnaht miteinander verbunden, insbesondere vernäht werden. Die der Anpassung an die Form der Körperextremität dienende Wölbung ist dabei zweckmäßig im Bereich und symmetrisch um die Längsnaht angeordnet. Zur Stabilisierung der Kompressionsbandage an der Körperextremität wird die Wölbung bevorzugt von zwei an der Außenseite des Bandagenkörpers aufgenähte bogenförmige Rippen umschlossen.

Vorteilhaft ist es, wenn die Anzahl der Befestigungslaschen der ersten Gruppe von Befestigungslaschen um eins größer ist als die Anzahl der Befestigungslaschen der zweiten Gruppe. Dadurch wird ermöglicht, dass die Einschnitte, welche die Befestigungslaschen der ersten Gruppe voneinander trennen, von den Befestigungslaschen der zweiten Gruppe (vollständig) überdeckt werden, so dass kein Körpergewebe durch diese Einschnitte hervorquellen kann, wenn die Kompressionsbandage an der Körperextremität angelegt ist.

Diese und weitere Vorteile und Merkmale der Erfindung ergeben sich aus dem nachfolgend unter Bezugnahme auf die begleitenden Zeichnungen näher beschriebenen Ausführungsbeispiel. Die Zeichnungen zeigen:
- **Fig. 1:**: Darstellung der Innenseite einer erfindungsgemäßen Kompressionsbandage;
- **Fig. 2:**: Darstellung der Außenseite der Kompressionsbandage von Fig. 1;
- **Fig. 3:**: Darstellung der Schritte zum Anlegen der Kompressionsbandage der Figuren 1 und 2 an einen Unterschenkel eines Patienten.

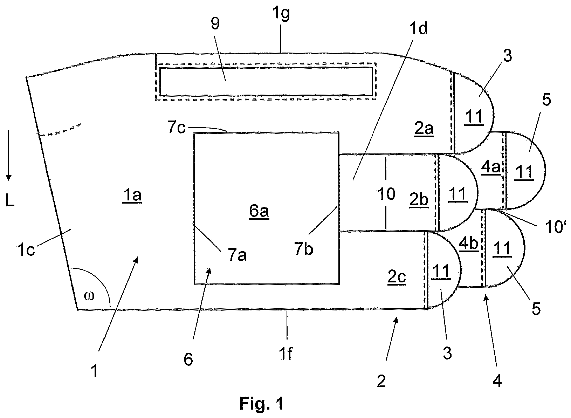

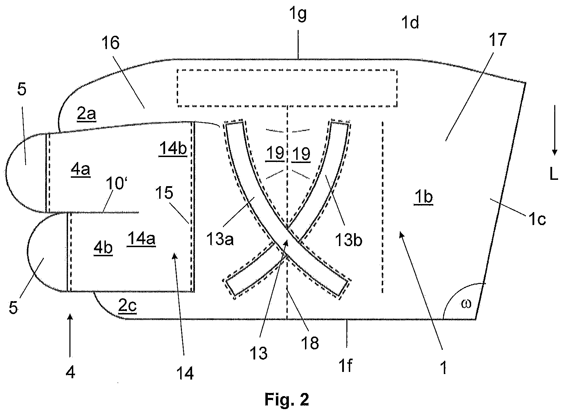

Die in den Figuren 1 und 2 jeweils in einer Draufsicht gezeigte Kompressionsbandage umfasst einen Bandagenkörper 1 aus einem elastischen Material. Bei dem elastischen Material kann es sich bspw. um ein Textilgewebe aus Polyamid und Elasthan, insbesondere mit 87% Polyamid und 13% Elasthan, bezogen auf das Gewicht des Textilgewebes handeln. Das elastische Material weist dabei eine materialspezifische Dehnungscharakteristik auf, die zweckmäßig eine maximale Dehnung des Materials im Bereich von 15 bis 50 % der ungedehnten Länge ermöglicht. Bevorzugt ist die maximale Dehnung entweder zwischen 15 und 20 % oder zwischen 25 und 35%.

Der Bandagenkörper 1 weist eine in Figur 1 sichtbare Innenseite 1a sowie eine in Figur 2 sichtbare Außenseite 1b auf. Der Bandagenkörper 1 umfasst dabei einen ersten Endabschnitt 1c und einen zweiten Endabschnitt 1d sowie eine distale Kante 1f und eine proximale Kante 1g. Der erste Endabschnitt 1c weist eine in Längsrichtung L des Bandagenkörpers 1 (von distal nach proximal) verlaufende glatte Kante auf, die zweckmäßig mit der distalen Kante 1f einen Winkel ω einschließt, der größer als 90° ist und insbesondere im Bereich von 100° bis 120° liegt. Am zweiten Endabschnitt 1d ist eine ersten Gruppe 2 von Befestigungslaschen angeordnet. Die Befestigungslaschen der ersten Gruppe 2 bestehen dabei zweckmäßig aus demselben elastischen Material wie der Bandagenkörper 1. Zweckmäßig sind die Befestigungslaschen 2a, 2b, 2c der ersten Gruppe 2 durch Einschnitte 10 in dem zweiten Endabschnitt 1d des Bandagenkörpers 1 ausgebildet und voneinander getrennt. Die Länge der Einschnitte 10 und damit die Länge der Befestigungslaschen der ersten Gruppe 2 ist dabei bevorzugt wesentlich kleiner als die Ausdehnung des Bandagenkörpers 1 in Querrichtung (Umfangsrichtung). Bevorzugt beträgt die Länge der Befestigungslaschen 2a - 2c der ersten Gruppe 2 von Befestigungslaschen zwischen 2cm und 5 cm.

In dem hier zeichnerisch dargestellten Ausführungsbeispiel einer erfindungsgemäßen Kompressionsbandage umfasst die erste Gruppe 2von Befestigungslaschen insgesamt drei Befestigungslaschen 2a, 2b, 2c, welche in Längsrichtung L des Bandagenkörpers 1 nebeneinander liegend angeordnet sind, wobei benachbarte Befestigungslaschen (2a, 2b; 2b, 2c) durch einen Einschnitt 10 in dem Bandagenkörper voneinander getrennt sind. Durch die Ausbildung der Befestigungslaschen 2a, 2b, 2c der ersten Gruppe 2 durch die Einschnitte 10 im zweiten Endabschnitt 1d des Bandagenkörpers 1 liegen benachbarte Befestigungslaschen 2a, 2b; 2b, 2c direkt nebeneinander, d.h. zwischen der Unterkante der oberen (proximalen) Befestigungslasche 2a und der Oberkante der sich distal daran anschließenden Befestigungslasche 2b liegt lediglich ein geringer Zwischenraum, der durch einen Einschnitt 10 gebildet ist. Entsprechend liegt zwischen der Oberkante der unteren (distalen) Befestigungslasche 2c und der Unterkante der sich in proximaler Richtung daran anschließenden Befestigungslasche 2b lediglich ein geringer Zwischenraum, der ebenfalls durch einen Einschnitt 10 gebildet ist.

Jede der Befestigungslaschen 2a, 2b, 2c der ersten Gruppe 2 von Befestigungslaschen verfügt über Befestigungsmittel 3, über welche die jeweilige Befestigungslasche 2a, 2b, 2c an der Außenseite 1b des Bandagenkörpers 1 befestigbar sind. Zweckmäßig sind die Befestigungsmittel 3 durch Klettverschlüsse gebildet, wobei Hakenteile der Klettverschlüsse an den äußeren (freien) Enden der Befestigungslaschen 2a, 2b, 2c angeordnet und die Außenseite 1b des Bandagenkörpers 1 mit Schlaufen zum Anhaften der Hakenteile 11 der Klettverschlüsse ausgebildet ist. Die Befestigungsmittel 3 können jedoch auch durch andere Mittel, wie z. B. Druckknöpfe, gebildet sein.

Wie aus Figur 2 ersichtlich ist auf der Außenseite 1b des Bandagenkörpers 1 eine zweite Gruppe 4 von Befestigungslaschen angeordnet. Diese zweite Gruppe 4 von Befestigungslaschen umfasst in dem hier zeichnerisch dargestellt Ausführungsbeispiel einer erfindungsgemäßen Kompressionsbandage zwei Befestigungslaschen 4a und 4b. Die Befestigungslaschen 4a, 4b, der zweiten Gruppe 4 sind dabei an einem Lachenband 14 angeordnet und erstrecken sich von einem ersten Endabschnitt 14a des Laschenbands 14. Die Befestigungslaschen 4a und 4b sind durch einen Einschnitt 10' im Laschenband 14 voneinander getrennt. Das Laschenband 14 und die daran angeordneten Befestigungslaschen der zweiten Gruppe 4 sind dabei zweckmäßig aus demselben elastischen Material gebildet wie der Bandagenkörper 1. Ein zweiter Endabschnitt 14b des Laschenbands 14 weist eine glatte Kante auf und ist an dieser Kante auf der Außenseite 1b des Bandagenkörpers 1 befestigt, bspw. durch eine Naht 15.

Die Befestigungslaschen 4a, 4b der zweiten Gruppe 4 von Befestigungslaschen sind dabei so auf der Außenseite 1b des Bandagenkörpers 1 angeordnet, dass sie die Befestigungslaschen 2a - 2c der ersten Gruppe 2 von Befestigungslaschen überdecken, insbesondere im Bereich der Einschnitte 10, welche benachbarte Befestigungslaschen der ersten Gruppe 2 voneinander trennen. Dabei sind die Befestigungslaschen der ersten Gruppe 2 in Bezug auf die Befestigungslaschen der zweiten Gruppe 4 in Längsrichtung L des Bandagenkörpers 1 (von distal nach proximal) versetzt zueinander angeordnet, so dass bspw. ein Mittelabschnitt der proximalen Befestigungslasche 4a der zweiten Gruppe 4 über dem Einschnitt 10 zwischen der proximalen Befestigungslasche 2a der ersten Gruppe und der sich daran in distaler Richtung anschließenden Befestigungslasche 2b zu liegen kommt, wie aus Figur 1 ersichtlich.

Wie die Befestigungslaschen der ersten Gruppe 2 verfügen auch die Befestigungslaschen der zweiten Gruppe 4 jeweils über zweite Befestigungsmittel 5. Vorteilhaft sind auch die zweiten Befestigungsmittel 5 durch Klettverschlüsse ausgebildet und umfassen Hakenteile 11, welche jeweils am freien Ende der Befestigungslaschen 4a, 4b der zweiten Gruppe 4 befestigt und so angeordnet sind, dass sie mit der Außenseite 1b des Bandagenkörpers 1 zur Herstellung einer Klettverbindung verbunden werden können.

Die Länge der Befestigungslaschen 4a, 4b der zweiten Gruppe 4 (also deren Ausdehnung quer zur Längsrichtung L des Bandagenkörpers 1) ist durch die Länge der Einschnitte 10' definiert und liegt zweckmäßig im Bereich von 1/3 bis 1/2 der Länge des Laschenbands 14. Bevorzugt beträgt die Länge der Befestigungslaschen 4a, 4b der zweiten Gruppe 4 zwischen 5cm und 10 cm und ist damit etwas länger als die Länge der Befestigungslaschen der ersten Gruppe 2.

Auf der Außenseite 1b des Bandagenkörpers 1 ist, wie aus Fig. 2 ersichtlich, ein Stabilisierungselement 13 vorgesehen. Das Stabilisierungselement 13 ist bei dem hier zeichnerisch dargestellten Ausführungsbeispiel durch zwei an der Außenseite 1b des Bandagenkörpers 1 aufgenähte Rippen 13a, 13b gebildet. Die Rippen 13a, 13b sind dabei zweckmäßig bogenförmig ausgebildet und V-förmig oder - wie in Fig. 1 gezeigt - X-förmig gekreuzt zueinander angeordnet. Die Rippen 13a, 13b bestehen dabei aus einem Textil- oder einem Kunststoffmaterial, das zweckmäßig auf der Außenseite des Bandagenkörpers 1 aufgenäht ist. Die Rippen 13a, 13b können jedoch auch durch Verkleben oder Verschweißen an der Außenseite 1b des Bandagenkörpers 1 befestigt sein.

Wie weiterhin aus Figur 2 ersichtlich, setzt sich der Bandagenkörper 1 aus zwei Teilen 16, 17 zusammen, welche entlang einer Längsnaht 18 miteinander vernäht sind. Im Bereich der Längsnaht 18 weist der Bandagenkörper 1 zumindest im proximalen Abschnitt eine zur Außenseite 1b hin konvex ausgeformte Wölbung 19 auf. Die bogenförmigen Rippen 13a, 13b sind dabei so auf der Außenseite 1a des Bandagenkörpers 1 angeordnet, dass sie die Wölbung 19 umschließen. In dem hier zeichnerisch in Fig. 2 dargestellten Ausführungsbeispiel sind die Rippen 13a, 13b X-förmig gekreuzt zueinander angeordnet und umschließen im oberen Abschnitt des "X" die Wölbung 19.

An der Innenseite 1a des Bandagenkörpers 1 ist eine Anziehhilfe in Form eines schlauchförmigen Einsatzes 6 angeordnet, wie aus Figur 1 ersichtlich. Der schlauchförmige Einsatz 6 ist dabei durch ein elastisches Tuch 6a (Textilmaterial) gebildet, welches an der Innenseite 1a des Bandagenkörpers 1 befestigt ist, insbesondere durch Vernähen. Das Textilmaterial des schlauchförmigen Einsatzes 6 ist dabei wesentlich dünner ausgebildet als das elastische Material des Bandagenkörpers 1 und besteht zweckmäßig ebenfalls aus einer Mischung von Polyamid und Elasthan, bspw. mit 71% Polyamid und 29% Elasthan, bezogen auf das Gesamtgewicht des elastischen Materials des schlauchförmigen Einsatzes 6.

Das elastische Material des Bandagenkörpers 1 weist eine Dehnungscharakteristik mit einer maximalen Dehnung im Bereich von 10% bis 50% des ungedehnten Zustands und bevorzugt im Bereich von 15% bis 35% des ungedehnten Zustands auf.

Bei dem zeichnerisch in Fig. 1 dargestellten Ausführungsbeispiel ist der schlauchförmige Einsatz 6 durch einen nahtlosen Textilschlauch 6a gebildet, der entlang von Längsnähten 7a, 7b an der Innenseite 1a des Bandagenkörpers 1 angenäht ist. Die beiden Längsnähte 7a, 7b verlaufen dabei zumindest im Wesentlichen parallel und im Abstand zueinander in Längsrichtung L des Bandagenkörpers 1. Zweckmäßig ist die der Innenseite 1a des Bandagenkörpers zugewandte innere Hälfte des Textilschlauchs 6b zusätzlich an ihrer proximalen Kante entlang einer Umfangsnaht 7c mit der Innenseite 1a des Bandagenkörpers 1 vernäht. An der Innenseite 1a des Bandagenkörpers 1 ist bevorzugt zwischen der proximalen Kante 1g und der Umfangsnaht 7c ein Haftband 9 angeordnet, welches sich in Querrichtung (Umfangsrichtung) des Bandagenkörpers 1 erstreckt.

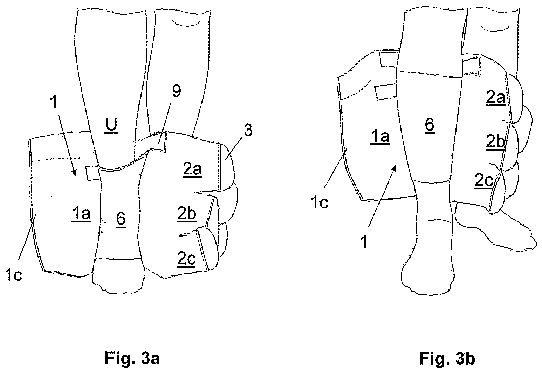

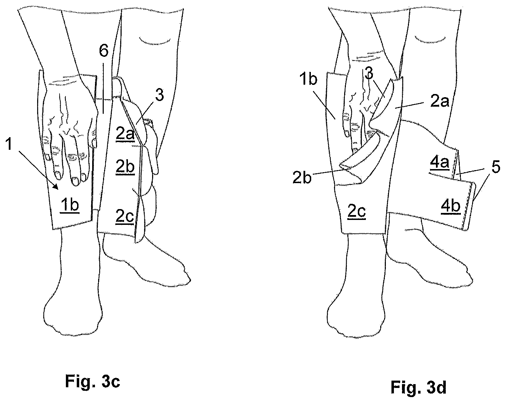

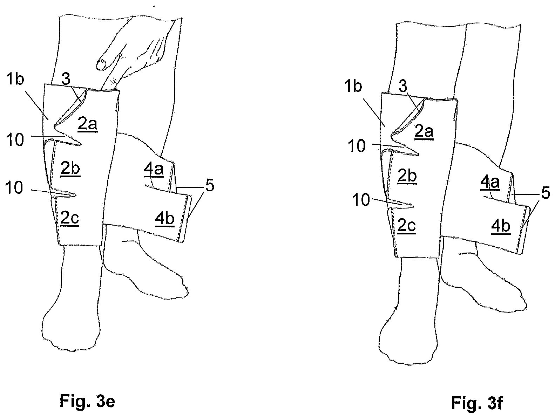

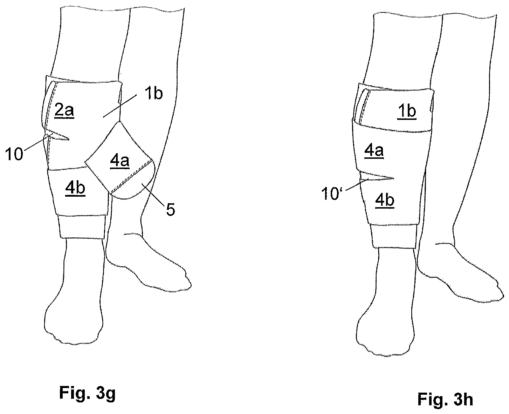

In Figur 3 sind schematisch die Schritte zum Anlegen der Kompressionsbandage an eine Körperextremität eines Patienten dargestellt, wobei es sich bei der Körperextremität beispielhaft um einen Unterschenkel U handelt. Zum Anlegen der Kompressionsbandage an den Unterschenkel U des Patienten wird zunächst der Bandagenkörper 1 um den Unterschenkel U geschlungen. Hierfür schlüpft der Patient mit seinem Unterschenkel U in den schlauchförmigen Einsatz 6 an der Innenseite 1a des Bandagenkörpers 1, bis die Wade im Bereich der Wölbung 19 des Bandagenkörpers 1 zu liegen kommt (Figuren 3a und 3b). Danach wird der laschenfreie erste Endabschnitt 1c des Bandagenkörpers 1 um eine Hälfte des Unterschenkels geschlungen und dort (auf der Außenseite des schlauchförmigen Einsatzes 6 aufliegend) festgehalten. Danach wird der zweite Endabschnitt 1d, an dem die Befestigungslaschen der ersten Gruppe 2 angeordnet sind, um die zweite Hälfte des Unterschenkels geschlungen (Figur 3c) und die Befestigungslaschen der ersten Gruppe 2 werden mittels der ersten Befestigungsmittel 3 auf der Außenseite 1b des Bandagenkörpers 1 befestigt, wie in Figur 3d gezeigt. Dabei wird durch Zug an den Befestigungslaschen 2 der ersten Gruppe eine Dehnung des Bandagenkörpers 1 bewirkt, welche durch die Befestigung der Befestigungslaschen auf der Außenseite 1b des Bandagenkörpers 1 mittels der ersten Befestigungsmittel 3 fixiert wird. Zweckmäßig werden die Befestigungslaschen 2a - 2c der ersten Gruppe 2 der Reihe nach, bspw. wie in den Figuren 3d und 3e gezeigt von distal nach proximal, auf der Außenseite 1b des Bandagenkörpers 1 befestigt. Nach dem Befestigen der letzten Befestigungslasche 2a der ersten Gruppe 2 können die Befestigungslaschen erforderlichenfalls noch einmal nachgezogen werden, indem sie - zweckmäßig wiederum der Reihe nach - kurz vom Bandagenkörper 1 gelöst und unter Zug an einer anderen Stelle des Bandagenkörpers wieder befestigt werden, um an dieser anderen Stelle einen größeren Zug am Bandagenkörper 1 auszuüben (Figur 3f). Der von den Befestigungslaschen der ersten gruppe 2 auf den Bandagenkörper 1 ausgeübte Zug bewirkt eine Dehnung des elastischen Materials des Bandagenkörpers 1, welche eine Kompression auf den Unterschenkel ausübt.

Zur Erhöhung der auf den Unterschenkel ausgeübten Kompression werden anschließend auch die Befestigungslaschen der zweiten Gruppe 4 auf der Außenseite 1b des Bandagenkörpers 1 befestigt. Hierfür wird wiederum zweckmäßig der Reihe nach, bspw. von proximal nach distal, jeweils an den Befestigungslaschen der zweiten Gruppe 4 gezogen und diese werden mittels der zweiten Befestigungsmittel 5 ebenfalls an der Außenseite 1b des Bandagenkörpers 1 befestigt (Figur 3g). Dabei werden die Befestigungslaschen der zweiten Gruppe 4 über die bereits am Bandagenkörper 1 befestigten Befestigungslaschen der ersten Gruppe 2 gelegt, so dass die Befestigungslaschen der zweiten Gruppe 4 die Befestigungslaschen der ersten Gruppe 2 überdecken, wie aus den Figuren 3g und 3h ersichtlich. Aufgrund der versetzten Anordnung der Befestigungslaschen der zweiten Gruppe 4 in Bezug auf die Befestigungslaschen der ersten Gruppe 2 werden dabei insbesondere die Einschnitte 10 zwischen benachbarten Befestigungslaschen 2a, 2b; 2b, 2c der ersten Gruppe 2 von den Befestigungslaschen der zweiten Gruppe 4 überdeckt. Dies verhindert, dass im Bereich der Einschnitte 10 geschwollenes Körpergewebe durchtreten kann. Durch den von den Befestigungslaschen der zweiten Gruppe 4 auf den Bandagenkörper 1 ausgeübten Zug wird der Bandagenkörper 1 noch weiter gedehnt, um eine noch höhere Kompression auf den Unterschenkel auszuüben. Durch den Ort der Befestigung der Befestigungslaschen 4a, 4b der zweiten Gruppe 4 auf der Außenseite 1b des Bandagenkörpers 1 kann der von der Kompressionsbandagen auf den Unterschenkel U ausgeübte Kompressionsdruck genau dosiert und eingestellt werden. Der von der Kompressionsbandage ausgeübte Kompressionsdruck ergibt sich durch die Gesamtdehnung des Bandagenkörpers 1, welche einerseits durch den Zug der Befestigungslaschen der ersten Gruppe 2 und den Zug der Befestigungslaschen der zweiten Gruppe 4 bewirkt wird.

Das auf der Außenseite 1b des Bandagenkörpers 1 angeordnete Stabilisierungselement 13 verhindert ein Verrutschen der angelegten Kompressionsbandage an der Körperextremität. Insbesondere wird durch das Stabilisierungselement 13 ein wulstförmiges Aufrollen des Bandagenkörpers 1 verhindert. Die Wölbung 19 im Zentralbereich des Bandagenkörpers 1 sorgt für eine anatomische Anpassung der Bandagenform an die Form der Körperextremität und verhindert damit ebenfalls, dass die angelegte Kompressionsbandage verrutschen kann. Die Wölbung 19 stellt weiterhin einen über den Umfang der Körperextremität gleichmäßig verteilten Kompressionsdruck sicher.

Die Erfindung ist nicht auf die hier zeichnerisch dargestellten Ausführungsformen beschränkt. So kann beispielsweise die Anzahl der Befestigungslaschen der ersten Gruppe von Befestigungslaschen bzw. der zweiten Gruppe von Befestigungslaschen an die Größe der Bandage angepasst werden. Bei längeren Bandagen kann bspw. die erste Gruppe 2 vier Befestigungslaschen umfassen und die zweite Gruppe 4 kann drei Befestigungslaschen umfassen. Zweckmäßig ist es dabei, wenn die Anzahl der Befestigungslaschen der ersten Gruppe 2 um eins größer ist als die Anzahl der Befestigungslaschen der zweiten Gruppe 4.

Die Dehnungscharakteristik der elastischen Materialen ist in weiten Bereichen variabel und kann den Erfordernissen der mit der Bandage durchzuführenden Kompressionsbehandlung angepasst werden. Weiterhin können statt Klettverschlüssen andere Befestigungsmittel zum Einsatz kommen, wie z.B. Druckknöpfe oder Haken- und Ösen-Verbindungen. Das zeichnerisch dargestellte Ausführungsbeispiel zweigt eine Bandage für einen rechten Unterschenkel. Für die Verwendung der Bandage für einen linken Unterschenkel werden die Endabschnitte 1c und 1d des Bandagenkörpers 1 zweckmäßig miteinander vertauscht. Dies gewährleistet, dass die Befestigungsmittel 3, 5 der ersten und zweiten Befestigungslaschen 2, 4 bei angelegter Bandage jeweils lateral auf der Außenseite des Bandagenkörpers 1 befestigt werden können. Die erfindungsgemäße Kompressionsbandage kann ferner auch an anderen Körperextremitäten angelegt werden, bspw. an einem Oberschenkel oder einem Unter- oder Oberarm.

## Patentansprüche

1. Kompressionsbandage zur Behandlung von Lymphödemen durch Anlegen der Kompressionsbandage an eine Körperextremität, wobei die Kompressionsbandage umfasst:
- einen Bandagenkörper (1) aus einem elastischen Material zum Umschlingen der Körperextremität, mit einer Innenseite (1a) und einer Außenseite (1b) sowie einem ersten und einem zweiten Endabschnitt (1c, 1d),
- eine erste Gruppe (2) von Befestigungslaschen, welche an einem Endabschnitt (1d) des Bandagenkörpers (1) angeordnet sind, wobei die Befestigungslaschen (2a, 2b, 2c) der ersten Gruppe (2) durch Einschnitte (10) in dem Bandagenkörper (1) voneinander getrennt sind und jeweils über erste Befestigungsmittel (3) verfügen, über welche die jeweilige Befestigungslasche (2a, 2b, 2c) der ersten Gruppe (2) an der Außenseite (1b) des Bandagenkörpers (1) befestigbar ist, wenn der Bandagenkörper (1) um die Körperextremität geschlungen ist,
- eine zweite Gruppe (4) von Befestigungslaschen, welche auf der Außenseite (1b) des Bandagenkörpers (1) befestigt und bei an der Körperextremität angelegter Kompressionsbandage so angeordnet sind, dass sie die Einschnitte (10) überdecken, welche die Befestigungslaschen (2a, 2b, 2c) der ersten Gruppe (2) voneinander trennen,
**dadurch gekennzeichnet, dass** die Befestigungslaschen (2a, 2b, 2c) der ersten Gruppe (2) an einem der beiden Endabschnitte (1d) des Bandagenkörpers (1) angeordnet sind und der andere Endabschnitt (1c) eine laschenfreie Außenkante aufweist.

2. Kompressionsbandage nach Anspruch 1, **dadurch gekennzeichnet, dass** die Befestigungslaschen (4a, 4b, 4c) der zweiten Gruppe (4) jeweils über zweite Befestigungsmittel (5) verfügen, über welche die jeweilige Befestigungslasche (4a, 4b, 4c) der zweiten Gruppe (4) an der Außenseite (1b) des Bandagenkörpers (1) befestigbar ist.

3. Kompressionsbandage nach Anspruch 1 oder 2, **dadurch gekennzeichnet, dass** die Kompressionsbandage auf die Körperextremität einen Kompressionsdruck ausübt, wenn der Bandagenkörper (1) um die Körperextremität geschlungen ist und die Befestigungslaschen der ersten und zweiten Gruppe (2, 4) an der Außenseite (1b) des Bandagenkörpers (1) befestigt sind, wobei der von der Kompressionsbandage auf die Körperextremität ausgeübte Kompressionsdruck durch die Positionierung der Befestigung der Befestigungslaschen an der Außenseite (1b) des Bandagenkörpers (1) einstellbar ist.

4. Kompressionsbandage nach einem der voranstehenden Ansprüche, **dadurch gekennzeichnet, dass** die ersten Befestigungsmittel (3) und/oder die zweiten Befestigungsmittel (5) Klettverschlüsse oder Hakenverschlüsse oder Druckknöpfe umfassen.

5. Kompressionsbandage nach einem der voranstehenden Ansprüche, **dadurch gekennzeichnet, dass** die ersten Gruppe (2) von Befestigungslaschen drei oder vier Befestigungslaschen (2a, 2b, 2c) umfasst und dass die zweite Gruppe (4) von Befestigungslaschen zwei oder drei Befestigungslaschen (4a, 4b) umfasst.

6. Kompressionsbandage nach einem der voranstehenden Ansprüche, **dadurch gekennzeichnet, dass** die Befestigungslaschen (2a, 2b, 2c) der ersten Gruppe (2) von Befestigungslaschen in Längsrichtung (L) des Bandagenkörpers (1) nebeneinander liegend angeordnet sind und dass die Befestigungslaschen (4a, 4b) der zweiten Gruppe (4) von Befestigungslaschen in Längsrichtung (L) des Bandagenkörpers (1) nebeneinander liegend angeordnet sind.

7. Kompressionsbandage nach einem der voranstehenden Ansprüche, **dadurch gekennzeichnet, dass** die Befestigungslaschen der zweiten Gruppe (4) in Längsrichtung (L) des Bandagenkörpers (1) versetzt zu den Befestigungslaschen der ersten Gruppe (2) angeordnet sind.

8. Kompressionsbandage nach einem der voranstehenden Ansprüche, **dadurch gekennzeichnet, dass** die Befestigungslaschen der zweiten Gruppe (4) an einem Laschenband (14) angeordnet sind und sich von einem ersten Endabschnitt (14a) des Laschenbands (14) erstrecken.

9. Kompressionsbandage nach Anspruch 8, **dadurch gekennzeichnet, dass** ein zweiter Endabschnitt (14b) des Laschenbands (14) an der Außenseite (1b) des Bandagenkörpers (1) befestigt, insbesondere vernäht ist.

10. Kompressionsbandage nach einem der voranstehenden Ansprüche, **dadurch gekennzeichnet, dass** an der Innenseite (1a) des Bandagenkörpers (1) eine Anziehhilfe in Form eines schlauchförmigen Einsatzes (6) angeordnet ist, wobei der schlauchförmige Einsatz (6) aus einem elastischen Textilmaterial gebildet und an der Innenseite (1a) des Bandagenkörpers (1) befestigt, insbesondere angenäht ist.

11. Kompressionsbandage nach einem der voranstehenden Ansprüche, **dadurch gekennzeichnet, dass** der Bandagenkörper (1) eine distale Kante (1f) und eine proximale Kante (1g) aufweist, wobei im Bereich der proximalen Kante (1g) an der Innenseite (1a) des Bandagenkörpers (1) wenigstens ein Haftband (9) angeordnet ist.

12. Kompressionsbandage nach einem der voranstehenden Ansprüche, **dadurch gekennzeichnet, dass** an der Außenseite (1b) des Bandagenkörpers (1) ein Stabilisierungselement (13) angeordnet ist.

13. Kompressionsbandage nach Anspruch 12, **dadurch gekennzeichnet, dass** das Stabilisierungselement (13) durch zwei an der Außenseite (1b) des Bandagenkörpers (1) aufgenähte und insbesondere bogenförmige Rippen (13a, 13b) gebildet ist, welche sich V- oder X-förmig kreuzen.

14. Kompressionsbandage nach Anspruch 13, **dadurch gekennzeichnet, dass** die Rippen (13a, 13b) aus einem Textilmaterial oder einem Kunststoffmaterial sind.

## Claims

1. Compression bandage for treating lymphatic edema by applying the compression bandage on a body extremity, wherein the compression bandage comprises:
- a bandage body (1) made of a resilient material to be wrapped around the body extremity, with an inner side (1a) and an outside (1b), as well as with a first and a second end section (1c, 1d),
- a first group (2) of fastening straps, which are arranged on an end section (1d) of the bandage body (1), wherein the fastening straps (2a, 2b, 2c) of the first group (2) are separated from one another by notches (10) in the bandage body (1) and have first fastening means (3), respectively, by means of which the respective fastening strap (2a, 2b, 2c) of the first group (2) can be fastened to the outside (1b) of the bandage body (1), when the body bandage (1) is wrapped around the body extremity,
- a second group (4) of fastening straps which are fastened to the outside (1b) of the bandage body (1) and, when the compression body is applied on the body extremity, are arranged so that they cover the notches (10) which separate the fastening straps (2a, 2b, 2c) of the first group (2) from one another,
**characterized in that** the fastening straps of the first group (2) are arranged on one of the two end sections (1d) of the bandage body (1), and the other end section (1c) comprises a strap-free outer edge.

2. Compression bandage according to Claim 1, **characterized in that** the fastening straps (4a, 4b, 4c) of the second group (4) each have second fastening means (5), by means of which the respective fastening strap (4a, 4b, 4c) of the second group (4) can be fastened to the outside (1b) of the bandage body (1).

3. Compression bandage according to Claim 1 or 2, **characterized in that** the compression bandage exerts a compression pressure on the body extremity, when the bandage body (1) is wrapped around the body extremity, and the fastening straps of the first and second group (2, 4) are fastened to the outside (1b) of the bandage body (1), wherein the compression pressure exerted by the compression bandage on the body extremity can be adjusted by positioning the fastening of the fastening straps on the outside (1b) of the bandage body (1).

4. Compression bandage according to one of the preceding Claims, **characterized in that** the first fastening means (3) and/or the second fastening means (5) comprise hook- and-loop fasteners and hook closures or snap fasteners.

5. Compression bandage according to one of the preceding Claims, **characterized in that** the first group (2) of fastening straps comprises three or four fastening straps (2a, 2b, 2c), and **in that** the second group (4) of fastening straps comprises two or three fastening straps (4a, 4b).

6. Compression bandage according to one of the preceding Claims, **characterized in that** the fastening straps (2a, 2b, 2c) of the first group (2) of fastening straps are arranged lying next to one another in the longitudinal direction (L) of the bandage body (1), and **in that** the fastening straps (4a, 4b) of the second group (4) of fastening straps are arranged lying next to one another in longitudinal direction (L) of the bandage body (1).

7. Compression bandage according to one of the preceding Claims, **characterized in that** the fastening straps of the second group (4) are arranged offset in the longitudinal direction (L) of the bandage body (1) with respect to the fastening straps of the first group (2).

8. Compression bandage according to one of the preceding Claims, **characterized in that** the fastening straps of the second group (4) are arranged on a lashing strap (14) and extend from a first end section (14a) of the lashing strap (14).

9. Compression bandage according to Claim 8, **characterized in that** a second end section (14b) of the lashing strap (14) is fastened, specifically sewn, on the outside (1b) of the bandage body (1).

10. Compression bandage according to one of the preceding Claims, **characterized in that** a pulling strap in the form of a tubular insert (6) is arranged on the inner side (1a) of the bandage body (1), wherein the tubular insert (6) is formed from a resilient textile material and fastened, specifically sewn, on the inner side (1a) of the bandage body (1).

11. Compression bandage according to one of the preceding Claims, **characterized in that** the bandage body (1) is having a distal edge (1f) and a proximal edge (1g), wherein in the region of the proximal edge (1g) an adhesive tape (9) is arranged on the inner side (1a) of the bandage body (1).

12. Compression bandage according to one of the preceding Claims, **characterized in that** a stabilization element (13) is arranged on the outside (1b) of the bandage body (1).

13. Compression bandage according to Claim 12, **characterized in that** the stabilization element (13) is formed by two, in particular arcuate, ribs (13a, 13b) sewn on the outside (1b) of the bandage body (1), which are crossing each other in the form of a V or X.

14. Compression bandage according to Claim 13, **characterized in that** the ribs (13a, 13b) are made from a textile or a plastic material.

## Revendications

1. Bandage de compression pour le traitement de lymphoedèmes par le placement du bandage de compression au niveau d'une extrémité corporelle, dans lequel le bandage de compression comprend :
-- un corps de bandage (1) en un matériau élastique pour entourer l'extrémité corporelle, avec un côté intérieur (1a) et un côté extérieur (1b) ainsi qu'une première et une deuxième section terminale (1c, 1d),
-- un premier groupe (2) de pattes de fixation qui sont disposées au niveau d'une section terminale (1d) du corps de bandage (1), dans lequel les pattes de fixation (2a, 2b, 2c) du premier groupe (2) sont séparées les unes des autres par des échancrures (10) dans le corps de bandage (1) et disposent respectivement de premiers moyens de fixation (3) par l'intermédiaire desquels les pattes de fixation (2a, 2b, 2c) respectives du premier groupe (2) peuvent être fixées au niveau du côté extérieur (1b) du corps de bandage (1), lorsque le corps de bandage (1) est enroulé autour de l'extrémité corporelle,
-- un deuxième groupe (4) de pattes de fixation qui sont fixées sur le côté extérieur (1b) du corps de bandage (1) et sont disposées sur le bandage de compression placé au niveau de l'extrémité corporelle de façon à ce qu'elles recouvrent les échancrures (10) qui séparent les unes des autres les pattes de fixation (2a, 2b, 2c) du premier groupe (2),
**caractérisé en ce que** les pattes de fixation (2a, 2b, 2c) du premier groupe (2) sont disposées au niveau de l'une des deux sections terminales (1d) du corps de bandage (1) et l'autre section terminale (1c) présente un bord extérieur sans patte.

2. Bandage de compression selon la revendication 1, **caractérisé en ce que** les pattes de fixation (4a, 4b, 4c) du deuxième groupe (4) disposent respectivement de deux moyens de fixation (5) par lesquels la patte de fixation (4a, 4b, 4c) respective du deuxième groupe (4) peut être fixée au niveau du côté extérieur (1b) du corps de bandage (1).

3. Bandage de compression selon la revendication 1 ou 2, **caractérisé en ce que** le bandage de compression exerce une pression de compression sur l'extrémité corporelle lorsque le corps de bandage (1) est enroulé autour de l'extrémité corporelle et les pattes de fixation du premier et du deuxième groupe (2, 4) sont fixées au niveau du côté extérieur (1b) du corps de bandage (1), dans lequel la pression de compression exercée par le bandage de compression sur l'extrémité corporelle peut être ajustée par le positionnement de la fixation des pattes de fixation au niveau du côté extérieur (1b) du corps de bandage (1).

4. Bandage de compression selon l'une des revendications précédentes, **caractérisé en ce que** le premier moyen de fixation (3) et/ou le deuxième moyen de fixation (5) comprennent des fermetures velcro ou des fermetures à crochets ou des boutonspression.

5. Bandage de compression selon l'une des revendications précédentes, **caractérisé en ce que** le premier groupe (2) de pattes de fixation comprend trois ou quatre pattes de fixation (2a, 2b, 2c) et **en ce que** le deuxième groupe (4) de pattes de fixation comprend deux ou trois pattes de fixation (4a, 4b).

6. Bandage de compression selon l'une des revendications précédentes, **caractérisé en ce que** les pattes de fixation (2a, 2b, 2c) du premier groupe (2) de pattes de fixation sont disposées côte à côte dans une direction longitudinale (L) du corps de bandage (1) et **en ce que** les pattes de fixation (4a, 4b) du deuxième groupe (4) de pattes de fixation sont disposées côte à côte dans une direction longitudinale (L) du corps de bandage (1).

7. Bandage de compression selon l'une des revendications précédentes, **caractérisé en ce que** les pattes de fixation du deuxième groupe (4) sont placées de façon décalée en direction longitudinale (L) du corps de bandage (1) par rapport aux pattes de fixation du premier groupe (2).

8. Bandage de compression selon l'une des revendications précédentes, **caractérisé en ce que** les pattes de fixation du deuxième groupe (4) sont disposées au niveaud'une bande de pattes (14) et s'étendent depuis une première section terminale (14a) de la bande de pattes (14).

9. Bandage de compression selon la revendication 8, **caractérisé en ce qu'**une deuxième section terminale (14b) de la bande de pattes (14) est fixée, en particulier est cousue, au niveau du côté extérieur (1b) du corps de bandage (1).

10. Bandage de compression selon l'une des revendications précédentes, **caractérisé en ce qu'**au niveau du côté intérieur (1a) du corps de bandage (1) est disposé un auxiliaire d'enfilage sous la forme d'un prolongement tubulaire (6), dans lequel le prolongement tubulaire (6) est formé d'un matériau textile élastique et est fixé, en particulier est cousu au niveau du côté intérieur (1a) du corps de bandage (1).

11. Bandage de compression selon l'une des revendications précédentes, **caractérisé en ce que** le corps de bandage (1) présente un bord distal (1f) et un bord proximal (1g), dans lequel dans la zone du bord proximal (1g) au niveau du côté intérieur (1a) du corps de bandage (1) est disposée au moins une bande adhésive (9) .

12. Bandage de compression selon l'une des revendications précédentes, **caractérisé en ce qu'**au niveau du côté extérieur (1b) du corps de bandage (1) est disposé un élément de stabilisation (13).

13. Bandage de compression selon la revendication 12, **caractérisé en ce que** l'élément de stabilisation (13) est formé par deux côtes (13a, 13b) cousues au niveau ducôté extérieur (1b) du corps de bandage (1) et en particulier, arquées, qui se croisent en formant un V ou un X.

14. Bandage de compression selon la revendication 13, **caractérisé en ce que** les côtes (13a, 13b) sont en un matériau textile ou en une matière plastique.
